# EUROPEAN PATENT SPECIFICATION

(11) **EP 1 750 769 B1**
(45) Date of publication and mention of the grant of the patent: **23.01.2013**
(21) Application number: 05771131.9
(22) Date of filing: 19.05.2005
(51) Int. Cl.: A61K 47/36

(54) **METHODS FOR MAKING INJECTABLE POLYMER HYDROGELS**
VERFAHREN ZUR HERSTELLUNG EINES INJIZIERBAREN POLYMERHYDROGELS
PROCEDE DE PREPARATION D'HYDROGELS POLYMERES INJECTABLES

(30) Priority: 20.05.2004 US 572944 P
(43) Date of publication of application: 14.02.2007
(73) Proprietor: Mentor Worldwide LLC, New Brunswick, NJ 08933 (US)
(72) Inventor: WANG, Wei, Aberdeen AB12 3QG (GB)
(74) Representative: Carpmaels & Ransford
(86) International application number: PCT/US2005/017641
(87) International publication number: WO 2005/112888

(56) References cited:
- FR-A- 2 819 722
- US-A- 4 716 154
- US-A1- 2002 035 168
- US-A1- 2003 148 995
- US-B1- 6 685 963

## Description

### TECHNICAL FIELD

This document relates to processes for preparing injectable polymer hydrogels.

### BACKGROUND

Injectable gels often are used for soft tissue augmentation. For example, injectable gels and be used as facial fillers for wrinkles and folds, lip enhancement and body contour correction, as well as in arthritis prostheses. Biocompatible polymers such as alginate acid, chitosan, polyacrylamide, and hyaluronan (hyaluronic acid, HA) have been used to prepare injectable gels for various applications. Injectable gels often are prepared by covalently crosslinking polymers in solution to form a rubber-like network structure, which is then mechanically homogenized to form injectable microparticles. Typically, each operation of these multi-step processes is separated, involving various pieces of equipment and product transfers.

US2003/0148995 discloses a process for the preparation of an injectable hydrogel, comprising the steps of hydrating hyaluronic acid with NaOH to form a gel of uncrosslinked NaHa polymer, crosslinking the polymer with BDDE in a water bath at 50°C +- 2°C for 1 h 45 min to 2 h 30 min, washing, purifying the gel in phosphate buffer to produce the hydrogel, and homogenizing the hydrogel.

### SUMMARY

This document provides simple, rapid and low cost processes for preparation of injectable hydrogels (e.g., injectable hyaluronan hydrogels). The processes include the steps of crosslinking one or more polymers and washing the subsequently formed gel, followed by purification and homogenization to produce an injectable hydrogel. The processes are carried out in a single reaction vessel as continuous processes, and thus result in elimination of the need to carry out any product transfer. In addition, no organic solvent or drying step is required. The processes also can provide an easily controllable and repeatable operation for very quick and low cost production of injectable gels, with different polymer concentrations and different particle sizes for various applications. One production cycle may take as little as three days.

Also described herein are hydrogels made by the processes described herein. The hydrogels can have a high degree of cross linking but a very deformable soft structure and superior biostability. As such, the gels can be used in soft tissue augmentation and medical prostheses. The swelling degree of the gels in PBS can be about 4000-5000%. The gels can have particle sizes on the order of 500 micrometers, and can be easily injected through G30½ needles (inner diameter 150 micrometer). Injectable hyaluronan gels produced by the processes provided herein can have superior viscoelasticity. The elastic modulus G*'* can be much higher than the viscous modulus G", the complex viscosity can be from about 2 x 10⁴ Pa.s to 35 Pa.s, and the phase angle delta (δ) can be very low (around 10), over a range of 0.01-10 Hz. In addition, the injectable hyaluronan gels prepared by the processes provided herein can exhibit a large degree of biostability to hyaluronidase as compared with injectable hyaluronan gels such as Restylane^{®} (Medicis Aesthetics, Inc., Scottsdale, AZ) and Hylaform^{®} (Inamed Aesthetics, Santa Barbara, CA).

In one aspect, this document features a process for the preparation of an injectable hydrogel. The process can include the steps of crosslinking one or more polymers to form a gel, washing the gel, purifying the gel, and homogenizing the gel to produce the hydrogel, wherein the process is carried out in a single reaction vessel as a continuous process. The polymer can have one or more reactive groups selected from hydroxyl groups, carboxyl groups and amine groups. The polymer is a polysaccharide selected from hyaluronic acid, chitosan, alginate acid, starch, dextran, or salts or water soluble derivatives thereof.

The crosslinking reaction can be carried out with a bi- or polyfunctional crosslinking agent, such as an epoxide, aldehyde, polyaziridyl or divinyl sulphone. The crosslinking agent can be 1,4-butanediol diglycidyl ether (BDDE). The process can be carried out at a pH of 11 or higher. The crosslinking reaction is carried out at a temperature of 37-60°C (e.g., 50°C), for at least 4 hours.

The process can further include preparing a solution of the polymer in NaOH and adding the crosslinking agent with stirring. The process can further include cutting the formed gel into pieces using one or more impellers in the reaction vessel, and washing and purifying the gel with one or more changes of PBS solution. The washing and purifying process can be carried out over 2 to 3 days with at least six changes of PBS solution.

The polymer can be hyaluronic acid. The process can be carried out with a solution of hyaluronic acid in 0.25 M NaOH, at a concentration up to 20% by weight. The initial concentration of hyaluronic acid can be 11-14% by weight. The molar ratio of crosslinking agent to polymer can be 0.5-2.4.

In another aspect, this document features an injectable hydrogel produced using a process described herein. In addition, this document features a biomaterial containing an injectable hydrogel as described herein. The biomaterial can be in the form of a sheet, bead, sponge, or formed implant.

Unless otherwise defined, all technical and scientific terms used herein have the same meaning as commonly understood by one of ordinary skill in the art to which this invention pertains. Although methods and materials similar or equivalent to those described herein can be used to practice the invention, suitable methods and materials are described below. In case of conflict, the present specification, including definitions, will control. In addition, the materials, methods, and examples are illustrative only and not intended to be limiting.

The details of one or more embodiments of the invention are set forth in the accompanying drawings and the description below. Other features, objects, and advantages of the invention will be apparent from the description and drawings, and from the claims.

### DESCRIPTION OF DRAWINGS

FIG. 1 is a drawing a stirrer vessel suitable for use in the continuous processes for preparing injectable hyaluronan gels as described herein.
FIG 2 is a graphical representation of the rheological data described in Example 1.

### DETAILED DESCRIPTION

Hyaluronan is a naturally occurring polysaccharide containing alternating N-acetyl-D-glucosamine and D-glucuronic acid monosaccharide units. As used herein "hyaluronan" refers to hyaluronic acid and its hyaluronate salts, including, but not limited to, sodium hyaluronate, potassium hyaluronate, magnesium hyaluronate and calcium hyaluronate.

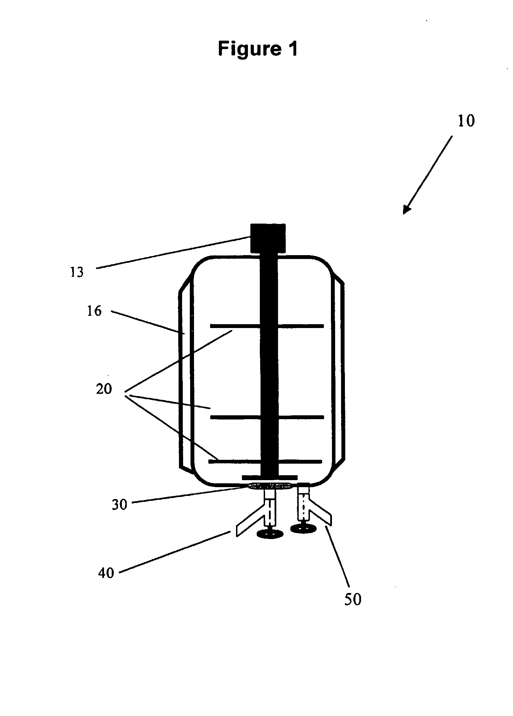

The methods provided herein can include the use of a vessel such as stirrer vessel 10 (Figure 1), which can be equipped with motor 13, jacket 16, and stirrers/impellers 20. Water, NaOH, and hyaluronan can be added into the vessel and stirred. The initial hyaluronan solution concentration, typically up to 20%, can be important in determining the properties of the final gel. An initial hyaluronan concentration of 11-14% (*e.g*., 11 %, 11.5%, 12%, 12.5%, 13%, 13.5%, or 14%) by weight can be particularly useful. If the hyaluronan solution concentration is lower (e.g., 8% or less), only a weak hydrogel may be obtained. Higher initial hyaluronan concentrations may result in hydrogels with too large a degree of crosslinking, which in turn can be difficult to homogenize to form injectable gels and which also may have poor viscoelasticity.

NaOH at a concentration of about 0.2 M to about 0.3 M (e.g., 0.2 M, 0.21 M, 0.22 M, 0.23 M, 0.24 M, 0.25 M, 0.26 M, 0.27 M, 0.28 M, 0.29 M, or 0.3 M) can be useful for dissolving hyaluronan quickly. Further, the inventors have found that crosslinking reactions can readily proceed at a pH higher than 11 (*e.g*., 11, 11.2, 11.4, 11.6, 11.8, 12, or higher than 12). In a typical process, 3.5 to 4 hours may be required to dissolve hyaluronan in 0.25 M NaOH at room temperature to produce a homogeneous solution, even at a concentration up to 20%.

A crosslinking agent such as, for example, 1,4-butanediol diglycidyl ether (BDDE) can be added and the hyaluronan solution is kept at a temperature between about 37°C and about 60°C (*e.g*., 37°C, 40°C, 45°C, 50°C, 55°C, or 60°C) for at least 4 hours. A temperature of 50°C and a reaction time of 4 hours can be particularly useful. At room temperature, no strong crosslinking is achieved, and at temperatures over 65°C the hyaluronan can degrade quickly. Shorter times such as 2 hours may not give strongly crosslinked gels, and longer times do not appear to provide gels with improved properties, but may result in degradation of the hyaluronan. The molar ratio of crosslinking agent, *e.g*., BDDE, to hyaluronan also can be an important parameter. A useful molar ratio can be in the range of 1.4:1 to 2.0:1 (*e.g*., 1.4:1, 1.5:1, 1.6:1, 1.7:1, 1.8:1, 1.9:1, or 2.0:1). These conditions can result in injectable gels having hyaluronan content of 19-23 mg/g and having very good viscoelasticity, injectability and biostability.

A crosslinking reaction can be stopped by lowering the temperature and PBS can be added to the formed hydrogel. The formed hydrogel can be exhaustively washed and purified directly with PBS (pH = 7.4) under stirring at room temperature in the stirrer vessel to remove residual crosslinking agent and unreacted hyaluronan from the gel. The gel can then be homogenized into small, injectable pieces by operating the impellers at a high speed. The impellers can have sharpened blades and can be moved in a vertical plane, which can facilitate homogenization. The distance between the outer edge of the impeller blades and the inner wall of the vessel should be kept to a minimum. Particle size typically decreases and deformability increases with increasing stirring time. The particles can be washed with one or more (e.g., one, two, three, four, five, six, seven, eight, nine, or ten) changes of PBS over a period of 1 to 4 days (e.g., 1, 1.5, 2, 2.5, 3, 3.5, or 4 days) via a well fitted filter and valve in the vessel (e.g., filter 30 and valve 40 on the bottom of stirrer vessel 10, shown in Figure 1). Washing in PBS for about 2.5 days, with about 6 changes of fresh PBS in the stirrer vessel, can be useful to obtain a gel that is pure and is hydrated to an equilibrium weight or volume, such that further washing with PBS does not increase the weight or volume of the obtained gel. Saturated gels stabilized with PBS typically are highly swollen. Finally, a purified gel can be homogenized by high speed stirring of the impellers. The gel can be removed from the vessel (e.g., through valve 50 shown in Figure 1) and packaged (e.g., in vials or syringes) before or after sterilization (e.g., by autoclaving).

Using the processes described herein, injectable hyaluronan gels with hyaluronan concentrations of, for example, from 1.0% to 3.5% (e.g., 1.0%, 1.1%, 1.2%, 1.25%, 1.3%, 1.4%, 1.5%, 1.6%, 1.7%, 1.75%, 1.8%, 1.9%, 2.0%, 2.1%, 2.2%, 2.25%, 2.3%, 2.4%, 2.5%, 2.6%, 2.7%, 2.75%, 2.8%, 2.9%, 3.0%, 3.1%, 3.2%, 3.25%, 3.3%, 3.4%, or 3.5%) can be prepared. Gels with lower concentrations of hyaluronan may not be sufficiently stable. Higher concentrations of hyaluronan can offer good stability, but injectability through G30 needles may be poor, although such gels can be injected through G27 needles having an inner diameter of 200 micrometers. Concentrations in the range of 1.9-2.3%, *i.e*., 19-23 mg/g gel, can have sufficient stability and good injectability through a G30 needle.

If the initial concentrations of hyaluronan and crosslinking agent are the same from preparation to preparation, final gels (after washing and purification) of consistent hyaluronan content and swelling degree can be obtained. Thus, the hyaluronan concentration and degree of swelling of the final injectable gel can be controlled by means of controlling initial hyaluronan solution concentration and the molar ratio of crosslinking agent to hyaluronan. In addition, the viscoelasticity, injectability, and biostability of the final injectable gels can be controlled by crosslinking level and crosslinking density, which are mainly controlled by initial hyaluronan concentration and molar ratio of crosslinking agent to hyaluronan.

Viscoelasticity can be measured using, for example, a rheometer at room temperature. Injectability can be tested using G 30 ½ needles, and biostability can be evaluated by incubation in a hyaluronidase PBS solution at 37°C for 24 hours, followed by analysis of degraded glucuronic acid weight using a carbazole assay (Bitter and Muir, Analytical Biochemistry, 1962, 4:330). For example, the inventors have found that digestion in PBS solution with 22 units of hyaluronidase (1 gram injectable gel in 5 ml) at 37°C for 24 hours resulted in a weight loss of about 10 percent.

Injectable gels prepared by the processes described herein may be further processed to form a variety of biomaterials such as sheets, beads, sponges, and formed implants. The gels can be used in a variety of pharmaceutical, medical (including surgical) and cosmetic applications. Thus, they may for example be useful in promoting wound healing, *e.g*., as a dermal wound dressing. They may also be useful in preventing adhesion formation *e.g*., preventing tissue growth between organs following surgery. The crosslinked gels may also find application in the ophthalmic field, *e.g.,* for vitreous fluid replacement, as corneal shields for delivery of drugs to the eye, or as lenticules.

The crosslinked gels also may be useful in surgery, for example as solid implants for hard tissue augmentation *e.g.,* repair or replacement of cartilage or bone, or for soft tissue augmentation, as breast implants, or as coating for implants intended for long term use in the body, such as breast implants, catheters, cannulae, bone prostheses, cartilage replacements, mini pumps and other drug delivery devices, artificial organs and blood vessels, meshes for tissue reinforcement, etc. They may also be used as joint lubricants in the treatment of arthritis.

A further use for the injectable gels prepared by the methods provided herein can be in the delivery of therapeutically active agents including in any of the aforementioned applications. Therapeutically active agents may be chemotherapeutic agents or biologically active factors (*e.g*., cytokines) and include anti-inflammatory agents, antibiotics, analgesics, anaesthetics, *e.g*., lidocaine, wound healing promoters, cytostatic agents, immunostimulants, immunosuppressants, DNA and antivirals. Such therapeutically active factors may be bound, either physically or chemically, to the crosslinked gel using methods well known in the art.

The present invention will now be illustrated by the following examples, which are not intended to limit the invention as set forth in the claims.

### EXAMPLES

### Example 1

This example illustrates a procedure for making an injectable hyaluronan gel.

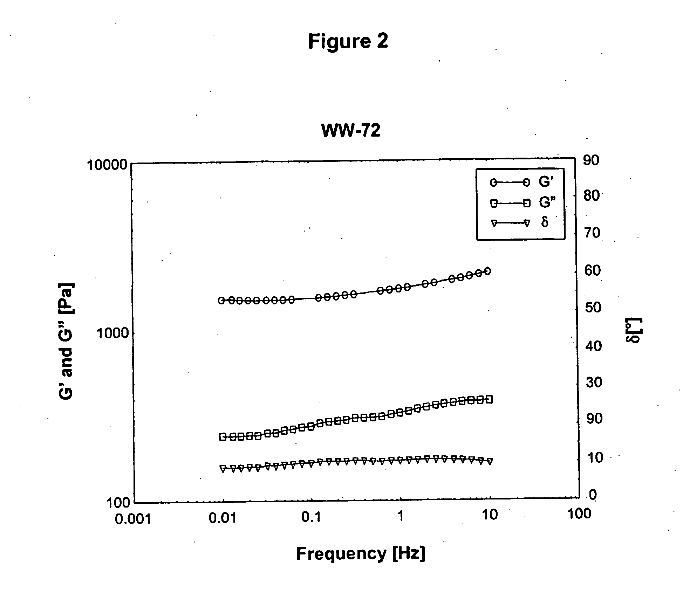

1.1 gram hyaluronan (MW: 2.3 × 10⁶) was dissolved in 10 ml 0.25 M NaOH aqueous solution in a stirrer vessel at room temperature for 4 hours. 1.0 ml BDDE was added to the hyaluronan solution under stirring, and then the solution was kept at 50°C for four hours. Subsequently 500 ml PBS was added to the stirrer vessel to wash and hydrate the obtained gel. After 2.5 day washing with six changes of fresh PBS, the gel was filtered to remove free PBS and stirred into injectable gel by impeller stirring for four hours. The yield of gel was 55 grams, which was then used to fill syringes for autoclaving. Figure 2 provides the rheological properties of prepared injectable gels. The percentage degraded by hyaluronidase was less than 10%.

### Example 2

This example illustrates the effect of initial hyaluronan solution concentration on rheology and biostability of the injectable gel.

Injectable gels were prepared with four hours crosslinking with BDDE at 50°C and BDDE/HA molar ratio of 1.4:1 and four hours homogenization, but using different initial hyaluronan solution concentrations. The properties of the gels produced are shown in Table 1.

It is clear that the concentration, viscoelasticity, and biostability of the gels was increased with increasing initial HA solution concentration, due to an increase in the level of crosslinking. An initial hyaluronan concentration of about 12% was found to be optimum, resulting in a gel with good viscoelasticity and biostability. Of course, other concentrations can be utilized according to the desired properties of the final gel.

**Table 1**

| *Effect of Initial Hyaluronan Concentration on Gel Concentration, Rheology and Biostability* | | | | |
|---|---|---|---|---|
| Initial HA solution concentration (g/ml) | 8% | 10% | 12% | 14% |
| Final gel concentration(mg/g) | 11 | 16 | 22 | 34 |

| Viscoelasticity(0.1-10 Hz) | | | | |
|---|---|---|---|---|
| G' (Pa) | 75-113 | 537-725 | 1324-1976 | Too dry to measure |
| G"(Pa) | 14-15 | 74-76 | 283-371 | |
| Phase angle | 10-8 | 8-6 | 12-10 | |
| Complex viscosity η*(Pa.s) | 95-2 | 950-12 | 1326-32 | |
| Biostability (degraded percent in hyaluronidase at 37°C for 24 hours) | 90 | 71 | 12 | 3.5 |

### Example 3

This example illustrates the effect of molar ratio of BDDE/hyaluronan on rheology and biostability of the injectable gel.

Injectable gels were prepared with four hour crosslinking with BDDE at 50°C and 10% initial hyaluronan concentration and four hour homogenization, but using different BDDE/HA molar ratios. The properties of the gels are given in Table 2.

The higher the molar ratio of BDDE/hyaluronan, the higher the gel concentration and viscoelasticity as well as the biostability, meaning that the degree of swelling decreased because of increasing crosslinking level and density.

**Table 2**

| *Effect of BDDE*/*HA molar ratio on Gel Concentration, Rheology and Biostability* | | | | |
|---|---|---|---|---|
| BDDE/HA molar ratio | 1.0:1 | 1.4:1 | 2.0:1 | 2.4:1 |
| Final gel concentration(mg/g) | 14 | 16 | 20 | 23 |

| Viscoelasticity(0.1-10 Hz) | | | | |
|---|---|---|---|---|
| G' (Pa) | 75-113 | 537-725 | 958-1254 | - |
| G"(Pa) | 14-15 | 74-76 | 153-166 | |
| Phase angle | 10-8 | 8-6 | 9-5 | |
| Complex viscosity η*(Pa.s) | 95-2 | 950-12 | 1212-20 | |
| Biostability (degraded percent in hyaluronidase at 37°C for 24 hours) | 81 | 71 | 29 | 23 |

### Example 4

This example illustrates the effect of crosslinking time on rheology and biostability of the injectable gel.

Injectable gels were prepared by crosslinking with BDDE at 50°C and with 10% initial hyaluronan concentration, a BDDE/hyaluronan molar ratio of 1.0:1 and four hour homogenization, but using differing crosslinking times. The properties of the gels are listed in Table 3.

Crosslinking level and density was increased with crosslinking reaction time. A four hour crosslinking reaction appeared to be optimum. Increasing this to five or six hours did not significantly change the properties of the resultant gels.

**Table 3**

| *Effect of Crosslinking Time on Gel Concentration, Rheology and Biostability* | | | | |
|---|---|---|---|---|
| Crosslinking time (hour) | 2 | 4 | 5 | 6 |
| Final gel concentration(mg/g) | 10 | 14 | 16 | 17 |

| Viscoelasticity (0.1-10 Hz) | | | | |
|---|---|---|---|---|
| G' (Pa) | 45-85 | 264-418 | 253-445 | 392-608 |
| G"(Pa) | 7-8 | 52-50 | 59-66 | 70-91 |
| Phase angle | 8-6 | 11-7 | 13-8 | 10-9 |
| Complex viscosity η*(Pa.s) | 56-2 | 337-7 | 326-7 | 498-10 |
| Biostability (degraded percent in hyaluronidase at 37°C for 24 hours) | Totally degraded | 81 | 79 | 76 |

### Example 5

This example illustrates the effect of stirring time on particle size of the injectable gel.

The gel prepared in Example 1 was homogenized for different times. The final particle size is shown in Table 4. A four hour homogenization was sufficient to provide good injectability through a G30 needle. The particle size was about 500 µm or lower.

**Table 4**

| *Effect of Homogenization Time on Particle Size and Injectability* | | | |
|---|---|---|---|
| Time (hours) | 2 | 3 | 4 |
| Particle Size (µm) | 610 | 550 | 510 |
| Injectability | Easy pass through G27 needle | | Easy pass through G30 needle |

### Example 6

This example illustrates the effect of crosslinking level on homogenization and particle size.

Gels were prepared at different initial HA concentrations and molar ratios of BDDE to hyaluronan. These were homogenized under the same conditions for four hours with a stirrer, with the resultant particle sizes being shown in Table 5. The lower the crosslinking level, the softer the gel obtained with resultant easier homogenization and smaller particle size.

**Table 5**

| *Effect of Crosslinking Level on Homogenization and Particle Size* | | | | |
|---|---|---|---|---|
| Initial HA concentration (%) | 10 | 10 | 10 | 11.5 |
| Molar ratio of BDDE/HA | 1.4:1 | 1.4:1 | 2.0:1 | 2.4:1 |
| Crosslinking time (hour) | 2 | 4 | 4 | 4 |
| Particle Size (µm) | 313 | 501 | 520 | 603 |
| Injectability through G30 ½ needle | Too easy | Easy | Little harder | Hard |

### Example 7

This example compares the properties of the gels made according to the processes described herein with commercial hyaluronan gels. The injectable gel prepared in Example 1 was analyzed and the properties evaluated and compared with Restylane^{®} and Hylaform^{®}, commercial soft tissue augmentation products. The results are shown in Table 6.

**Table 6**

| *Property Comparison of Injectable Gel with Restylane and Hylaform* | | | |
|---|---|---|---|
| Injectable gel | Gel prepared as described herein | Restylane^{®} | Hylaform^{®} |
| Gel concentration (mg/g) | 21 | 20 | - |

| Viscoelasticity (over the range of 0.1-10 Hz) | | | |
|---|---|---|---|
| G' (Pa) | 1559-2198 | 666-1042 | 114-173 |
| G" (Pa) | 274-384 | 132-183 | 23-22 |
| Phase angle | 9-10 | 11-10 | 11-8 |
| Complex viscosity η*(Pa.s) | 2519-35 | 1080-20 | 185-3 |
| Particle Size(µm) | 500 | 450 | 589 |
| Biostability (degraded percent in hyaluronidase at 37 °C for 24 hours) | About 10% | Over 90% | Over 60% |

### OTHER EMBODIMENTS

It is to be understood that while the invention has been described in conjunction with the detailed description thereof, the foregoing description is intended to illustrate and unot limit the scope of the invention, which is defined by the scope of the appended claims.

## Claims

1. A process for the preparation of an injectable hydrogel, the process comprising the steps of crosslinking one or more polymers to form a gel, washing the gel, purifying the gel, and homogenizing the gel to produce the hydrogel, wherein the process is carried out in a single reaction vessel as a continuous process, wherein the crosslinking reaction is carried out at a temperature of 37-60°C, for at least 4 hours, wherein the polymer is a polysaccharide, wherein the polysaccharide is hyaluronic acid, chitosan, alginate acid, starch, dextran, or salts or water soluble derivatives thereof.

2. A process as claimed in claim 1, wherein the crosslinking reaction is carried out with a bi- or polyfunctional crosslinking agent.

3. A process as claimed in claim 2, wherein the crosslinking agent is an epoxide, aldehyde, polyaziridyl or divinyl sulphone.

4. A process as claimed in claim 2, wherein the crosslinking agent is 1,4-butanediol diglycidyl ether (BDDE).

5. A process as claimed in any one of claims 1 to 4, which is carried out at a pH of 11 or higher.

6. A process as claimed in any one of claims 1 to 5, wherein the crosslinking reaction is carried out at a temperature of 50°C.

7. A process as claimed in any one of claims 5 to 6, wherein a solution of the polymer in NaOH is first prepared, to which is added the crosslinking agent, with stirring.

8. A process as claimed in any one of claims 1 to 7, wherein the formed gel is subsequently cut into pieces using one or more impellers in the reaction vessel and is then washed and purified by means of one or more changes of PBS solution.

9. A process as claimed in claim 8, wherein the washing and purification process is carried out over 2 to 3 days with at least six changes of PBS solution.

10. A process as claimed in any one of claims 1 to 9, wherein the polymer is hyaluronic acid.

11. A process as claimed in claim 10, wherein the process is carried out with a solution of hyaluronic acid in 0.25M NaOH, at a concentration up to 20%, by weight.

12. A process as claimed in claim 11, wherein the initial concentration of hyaluronic acid is 11-14%, by weight.

13. A process as claimed in any one of claims 1 to 12, wherein the molar ratio of crosslinking agent to polymer is 0.5-2.4.

## Patentansprüche

1. Verfahren zur Herstellung eines injizierbaren Hydrogels, bei dem man ein oder mehrere Polymere vernetzt, wobei man ein Gel erhält, das Gel wäscht, das Gel reinigt und das Gel homogenisiert, wobei man das Hydrogel erhält, wobei das Verfahren in einem einzigen Reaktionsgefäß als kontinuierliches Verfahren durchgeführt wird, wobei die Vernetzungsreaktion über einen Zeitraum von mindestens 4 Stunden bei einer Temperatur von 37-60°C durchgeführt wird, wobei es sich bei dem Polymer um ein Polysaccharid handelt, wobei es sich bei dem Polysaccharid um Hyaluronsäure, Chitosan, Alginsäure, Stärke, Dextran oder Salze oder wasserlösliche Derivate davon handelt.

2. Verfahren nach Anspruch 1, bei dem man die Vernetzungsreaktion mit einem bi- oder polyfunktionellen Vernetzungsmittel durchführt.

3. Verfahren nach Anspruch 2, bei dem es sich bei dem Vernetzungsmittel um ein Epoxid, einen Aldehyd, ein Polyaziridyl oder Divinylsulfon handelt.

4. Verfahren nach Anspruch 2, bei dem es sich bei dem Vernetzungsmittel um 1,4-Butandioldiglycidylether (BDDE) handelt.

5. Verfahren nach einem der Ansprüche 1 bis 4, das man bei einem pH-Wert von 11 oder mehr durchführt.

6. Verfahren nach einem der Ansprüche 1 bis 5, bei dem man die Vernetzungsreaktion bei einer Temperatur von 50°C durchführt.

7. Verfahren nach einem der Ansprüche 5 bis 6, bei dem man zunächst eine Lösung des Polymers in NaOH herstellt, die dann unter Rühren mit dem Vernetzungsmittel versetzt wird.

8. Verfahren nach einem der Ansprüche 1 bis 7, bei dem man das gebildete Gel anschließend unter Verwendung von einem oder mehreren Impellern im Rekationsgefäß in Stücke schneidet und dann mit Hilfe eines oder mehrerer Wechsel von PBS-Lösung wäscht und reinigt.

9. Verfahren nach Anspruch 8, bei dem man das Waschen und Reinigen über einen Zeitraum von 2 bis 3 Tagen mit mindestens sechs Wechseln von PBS-Lösung durchführt.

10. Verfahren nach einem der Ansprüche 1 bis 9, bei dem es sich bei dem Polymer um Hyaluronsäure handelt.

11. Verfahren nach Anspruch 10, bei dem man das Verfahren mit einer Lösung von Hyaluronsäure in 0,25 M NaOH bei einer Konzentration von bis zu 20 Gew.- % durchführt.

12. Verfahren nach Anspruch 11, bei dem die Anfangskonzentration von Hyaluronsäure 11-14 Gew.-% beträgt.

13. Verfahren nach einem der Ansprüche 1 bis 12, bei dem das Molverhältnis von Vernetzungsmittel zu Polymer 0,5-2,4 beträgt.

## Revendications

1. Procédé pour la préparation d'un hydrogel injectable, le procédé comprenant les étapes de réticulation d'un ou plusieurs polymères pour former un gel, lavage du gel, purification du gel, et homogénéisation du gel pour produire l'hydrogel, le procédé étant conduit dans une cuve de réaction unique sous la forme d'un procédé continu, la réaction de réticulation étant conduite à une température de 37 à 60 °C, pendant au moins 4 heures, le polymère étant un polysaccharide, le polysaccharide étant l'acide hyaluronique, le chitosane, l'acide alginique, l'amidon, le dextrane, ou des sels ou dérivés hydrosolubles de ceux-ci.

2. Procédé selon la revendication 1, dans lequel la réaction de réticulation est conduite avec un agent de réticulation bi- ou polyfonctionnel.

3. Procédé selon la revendication 2, dans lequel l'agent de réticulation est un époxyde, aldéhyde, polyaziridyle ou divinylsulfone.

4. Procédé selon la revendication 2, dans lequel l'agent de réticulation est l'éther diglycidylique de 1,4-butanediol (BDDE).

5. Procédé selon l'une quelconque des revendications 1 à 4, qui est conduit à un pH de 11 ou plus.

6. Procédé selon l'une quelconque des revendications 1 à 5, dans lequel la réaction de réticulation est conduite à une température de 50 °C.

7. Procédé selon l'une quelconque des revendications 5 à 6, dans lequel une solution du polymère dans du NaOH est préparée dans un premier temps, à laquelle est ajouté l'agent de réticulation, sous agitation.

8. Procédé selon l'une quelconque des revendications 1 à 7, dans lequel le gel formé est ensuite découpé en morceaux en utilisant un ou plusieurs propulseurs dans la cuve de réaction et est ensuite lavé et purifié au moyen d'un ou plusieurs changements de solution de PBS.

9. Procédé selon la revendication 8, dans lequel le processus de lavage et de purification est conduit en 2 à 3 jours avec au moins six changements de solution de PBS.

10. Procédé selon l'une quelconque des revendications 1 à 9, dans lequel le polymère est l'acide hyaluronique.

11. Procédé selon la revendication 10, dans lequel le procédé est conduit avec une solution d'acide hyaluronique dans du NaOH 0,25 M, à une concentration allant jusqu'à 20 %, en poids.

12. Procédé selon la revendication 11, dans lequel la concentration initiale d'acide hyaluronique est de 11 à 14 %, en poids.

13. Procédé selon l'une quelconque des revendications 1 à 12, dans lequel le rapport molaire de l'agent de réticulation au polymère est de 0,5 à 2,4.
